# EUROPEAN PATENT SPECIFICATION

(11) **EP 1 274 457 B1**
(45) Date of publication and mention of the grant of the patent: **30.11.2005**
(21) Application number: 01923433.5
(22) Date of filing: 09.04.2001
(51) Int. Cl.: A61K 45/06, A61P 11/02, A61P 37/00

(54) **METHOD AND COMPOSITIONS FOR THE TREATMENT OF ALLERGIC CONDITIONS USING PGD2 RECEPTOR ANTAGONISTS**
VERFAHREN UND ZUSAMMENSETZUNGEN ZUR BEHANDLUNG VON ALLERGISCHEN ZUSTÄNDEN MIT PGD2 REZEPTOR ANTAGONISTEN
PROCEDE ET COMPOSITIONS DESTINES AU TRAITEMENT D'ETATS ALLERGIQUES FAISANT APPEL A DES ANTAGONISTES DU RECEPTEUR PGD2

(30) Priority: 12.04.2000 US 196641 P
(43) Date of publication of application: 15.01.2003
(73) Proprietor: Merck Frosst Canada & Co., Kirkland, Quebec H9H 3L1 (CA)
(72) Inventor: JONES, Thomas, R., Kirkland, Québec H9H 3L1 (CA)
(74) Representative: Horgan, James Michael Frederic
(86) International application number: PCT/CA2001/000491
(87) International publication number: WO 2001/078697

(56) References cited:
- Y.IGARASHI E.A.: "Effects of inhibitors of arachidonic acid metabolism on serotonin release from rat basophilic leukemia cells" IMMUNOPHARMACOLOGY, vol. 25, no. 2, 1993, pages 131-144, XP001064118
- J.F.REGAL, R.L.BELL: "Mediators of C5a-induced bronchoconstriction in the guinea pig" INTERNATIONAL ARCHIVES OF ALLERGY AND APPLIED IMMUNOLOGY, vol. 84, no. 4, 1987, pages 414-423, XP001064252

## Description

### BACKGROUND OF THE INVENTION

Histamine, cysteinyl leukotrienes (CysLTs), prostaglandin D2 (PGD2) and thromboxane A₂ (TxA₂) are considered to be key mediators in allergic conditions such as allergic rhinitis and allergic conjunctivitis (Chan et al., 1989; Narita et al., 1996; Yamasaki et al., 1997; Yasui et al., 1997; Fujita et al., 1997). Released by activated mast cells they have been shown to increase microvascular permeability, blood flow, intranasal pressure and mucus secretion. These mediators assert their physiological effects primarily through interaction with their respective receptors; accordingly, treatments for allergic conditions have included agents that can block or otherwise interrupt such interactions. For example, anti-histamines and leukotriene D4 receptor antagonists have been shown previously to be effective in a guinea pig model of allergic rhinitis and conjunctivitis. (Chan et al., 1989). Leukotriene antagonists are now part of the arsenal for the treatment of asthma, and antihistamines have long been used to treat symptoms of allergic rhinitis. Because allergic conditions are attributed to multiple mediators, blocking the interaction of one mediator with its receptor may not be sufficient to alleviate the multitude of symptoms often associated with allergic conditions.

Thus, while antihistamines have been shown efficacious for preventing and relieving sneezing, itching, rhinorrhea and other symptoms of the early allergic response, they have not been found to be very effective for relief of the nasal blockage which is characteristic of the later stages of an allergic reaction. Thus, it has been common to concurrently administer sympathomimetic amine decongestant drugs, such as phenylpropanolamine or pseudoephedrine which function as alpha - adrenoceptor agonists; several combination products containing both antihistamine and sympathomimetic amine decongestants are commercially available. However, not all allergy sufferers should use these decongestant drugs, due to their frequently observed central nervous system and cardiovascular side effects which include agitation, sleeplessness, tachycardia, angina pectoris and hypertension. Recently, phenylpropanolamine was withdrawn from the US market.

It would be desirable to have available a treatment for allergic conditions which provides relief from all of the common symptoms thereof, particularly a treatment for allergic rhinitis that includes relief from nasal congestion, but which does not exhibit adverse nervous system or cardiovascular effects associated with sympathomimetic amines.

Prostaglandin D2 (PGD2) is also thought to be involved in human allergic rhinitis, a frequent allergic disease that is characterized by itch, sneezing, rhinorrhea and nasal congestion (Baraniuk, 1998; Doyle et al., 1990; Raphael et al., 1991; Ramis et al., 1991). Nasal provocation with PGD2 provoked a dose-dependent increase in nasal congestion, the most manifest symptom of allergic rhinitis (Doyle et al., 1990). In addition, elevated levels of PGD2 were noted in the nasal wash fluid of allergic patients that underwent a nasal antigen challenge.

Prostaglandin D2 antagonists said to be useful in the treatment of nasal occlusion have been disclosed in, for example, PCT Published Applications WO97/00853 and WO98/25919, and European Patent Applications EP945450 and EP944614.

### SUMMARY OF THE INVENTION

The present invention provides a combination for the treatment of allergic conditions, said combination comprising a prostaglandin D2 receptor antagonist and at least one other therapeutically active compound selected from histamine H1 receptor antagonists and leukotriene antagonists. The invention further provides pharmaceutical compositions containing a PGD2 antagonist and at least one other active ingredient selected from antihistamines and leukotriene antagonists.

### BRIEF DESCRIPTION OF THE FIGURES

FIG 1. summarizes the effect of the antihistamine mepyramine and compound I administered alone and in combination with each other on the changes in intranasal pressure induced by a nasal challenge of ovalbumin 1% for 3 minutes in ovalbumin-sensitized guinea pigs.
FIG 2. shows changes in nasal airway resistance (NAR) in allergic sheep following challenges with PGD2, leukotriene D4 (LTD4) and PGD2 + LTD4.

### DETAILED DESCRIPTION OF THE INVENTION

The present invention provides a combination for the treatment of allergic conditions which comprises an effective amount of a prostaglandin D2 receptor antagonist and an effective amount of at least one other therapeutically active compound selected from histamine H1 antagonists and leukotriene D4 receptor antagonists.

In another aspect, the present invention provides a pharmaceutical composition which comprises an effective amount of a PGD2 antagonist and at least one other therapeutically active compound selected from histamine H1 antagonists and leukotriene antagonists, and a pharmaceutically acceptable carrier.

As used herein the following terms have the indicated meanings:

The term "allergic conditions" means diseases or disorders associated with Type I hypersensitivity reactions, which are related or caused by antigen combining with IgE antibodies bound to receptors on mast cells. Examples of allergic conditions contemplated include allergic rhinitis (seasonal or perennial), allergic conjunctivitis, allergic asthma and urticaria.

The term "prostaglandin D2 receptor antagonist" (or PGD2 antagonist or DP antagonist) means compounds that are capable of blocking, inhibiting, reducing or otherwise interrupting the interaction between prostaglandin D2 and its receptor (eg DP receptor or other prostaglandin binding receptors such as CRTH2 receptors). The PGD2 antagonist may be selective (interact preferentially with) for the DP receptor or may possess antagonistic effects at one or more other prostaglandin receptors such as the thromboxane receptor (TP receptor) or other prostaglandin D2 binding receptors such as CRTH2 receptors.

The term "histamine H1 receptor antagonist" (or antihistamine) means any compounds that are capable of blocking, inhibiting, reducing or otherwise interrupting the interaction between histamine and its receptor.

The term "leukotriene D4 receptor antagonist" (or leukotriene antagonist or LTD4 antagonists) means any compounds that are capable of blocking, inhibiting, reducing or otherwise interrupting the interaction between leukotrienes and the Cys LT1 receptor.

The term "treatment" or "treating" includes alleviating, ameliorating, relieving or otherwise reducing, as well as preventing the onset of symptoms commonly associated with allergic conditions.

The term "effective amount" means that amount of the therapeutically active compound (PGD2 antagonist, antihistamine and leukotriene antagonist) which, alone or in combination, provides a therapeutic benefit in the treatment, management, or prevention of allergic conditions.

The term "composition", as in pharmaceutical composition, is intended to encompass a product comprising the active ingredients, and the inert ingredient(s) (pharmaceutically acceptable excipients) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a PGD2 antagonist and at least one other active ingredient selected from antihistamines and leukotriene antagonists, and pharmaceutically acceptable excipients.

Examples of PGD2 antagonists include, but are not limited to, compounds described as having PGD2 antagonizing activity in PCT Published Applications WO97/00853 and WO98/25919, and European Patent Applications EP945450 and EP944614, as well as the specific compounds 2-[(1R)-9-(4-chlorobenzyl)-8-((R)-methylsulfinyl)-2,3,4,9-tetrahydro-1H-carbazol-1-yl]acetic acid and 2-[(1R)-9-(4-chlorobenzyl)-8-((S)-methylsulfinyl)-2,3,4,9-tetrahydro-1H-carbazol-1-yl]acetic acid (Compound I).

Other PGD2 antagonists may be identified and evaluated using known methods including but not limited to, 1) radioligand binding assays using membranes from cells that express recombinant DP receptor, or platelet membranes, or membranes from cell lines and tissues that express endogenous DP, or 2) adenylyl cyclase assays using membranes from cells that express recombinant DP receptor or platelet membranes or membranes from cell lines and tissues that express endogenous DP, or 3) signal transduction assays using cells that express recombinant DP receptor or platelets or cells and tissues that endogenously express DP. Signal transduction assays may include but are not limited to cyclic AMP accumulation assays, protein kinase A activation assays and reporter-gene transcription based assays

Examples of antihistamines include, but are not limited to, azelastine, acrivastine, cyclizine, carebastine, cyproheptadine, carbinoxamine, doxylamine, dimethindene, ebastine, epinastine, efletirizine, ketotifen, levocabastine, mizolastine, mequitazine, mianserin, noberastine, meclizine, norastemizole, picumast, tripelenamine, temelastine, trimeprazine, triprolidine, bromopheniramine, chlorpheniramine, dexchlorpheniramine, triprolidine, clemastine, diphenhydramine, diphenylpyraline, tripelennamine, hydroxyzine, methdilazine, promethazine, trimeprazine, azatadine, cyproheptadine, antazoline, pheniramine, pyrilamine, astemizole, terfenadine, loratadine, cetirizine, levocetirizine, fexofenadine, descarboethoxyloratadine. Other compounds can readily be evaluated to determine activity at the H1 receptors by known methods, inluding specific blockade of the contractile response to histamine of isolated guinea pig ileum. Preferred anthistamines include loratadine, fexofenadine, cetirizine, descarboethoxyloratadine, astemizole, noraztemizole, and levocetirizine.

Examples of LTD4 antagonists include, but are not limited to, zafirlukast, montelukast, pranlukast, iralukast, pobilukast, SKB-106,203. Other compound can readily be evaluated to determine activity at the LTD4 receptors by known methods, including, but not limited to, those referenced or described in US Patent 5,565,473. Preferred leukotriene antagonists include montelukast, zafirlukast and pranlukast.

In one embodiment the present invention provides a combination useful in a method for treating allergic rhinitis which comprises administering to a patient in need of such treatment an effective amount of a PGD2 antagonist and an effective amount of an antihistamine.

In another embodiment the present invention provides a combination useful in a method for treating allergic rhinitis which comprises administering to a patient in need of such treatment an effective amount of a PGD2 antagonist and an effective amount of a leukotriene antagonist.

In yet another embodiment the present invention provides a pharmaceutical composition which comprises an effective amount of a PGD2 antagonist and an effective amount of an antihistamine.

In yet another embodiment the present invention provides a pharmaceutical composition which comprises an effective amount of a PGD2 antagonist and an effective amount of a leukotriene antagonist.

A further embodiment of the present invention provides a pharmaceutical composition which comprises an effective amount of a PGD2 antagonist, an effective amount of a leukotriene antagonist, and an effective amount of an antihistamine.

The PGD2 antagonist and the other active ingredient(s) may be administered in separate dosage forms, or all the active ingredients may be incorporated into a single dosage form. When administered in separate dosage forms, the various active compounds may be administered in any order, either simultaneously or sequentially. Furthermore, the separate dosage forms may each contain more than one active ingredient; for example a dosage form containing a PGD2 antagonist may be co-administered with a dosage form containing an antihistamine in combination with a LTD4 antagonist.

The dose of the active ingredients will vary with the nature and the severity of the condition to be treated and with the particular active ingredients chosen. It will also vary according to the age, weight and response of the individual patient. In general, the daily dose range for each active ingredient lies within the range of from about 0.001 mg to about 100 mg per kg body weight of a mammal, preferably 0.01 mg to about 50 mg per kg, in single or divided doses. On the other hand, it may be necessary to use dosages outside these limits in some cases.

Any suitable route of administration may be employed for providing a patient with an effective dosage of composition of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like.

The pharmaceutical compositions of the present invention comprise a PGD2 antagonist in combination with at least one other active ingredient selected from antihistamines and LTD4 antagonists, and a pharmaceutically acceptable carrier. The compositions include compositions suitable for oral, rectal, topical, parenteral (including subcutaneous, intramuscular, and intravenous), ocular (ophthalmic), pulmonary (aerosol inhalation), or nasal administration, although the most suitable route in any given case will depend on the nature and severity of the conditions being treated and on the nature of the active ingredient(s). They may be conveniently presented in unit dosage form and prepared by any of the methods well-known in the art of pharmacy.

For administration by inhalation, the therapeutically active ingredients compounds are conveniently delivered in the form of an aerosol spray presentation from pressurized packs or nebulisers. The compounds may also be delivered as powders which may be formulated and the powder composition may be inhaled with the aid of an insufflation powder inhaler device. The preferred delivery systems for inhalation are metered dose inhalation (MDI) aerosol, which may be formulated as a suspension or solution of the therapeutically active compounds in suitable propellants, such as fluorocarbons or hydrocarbons and dry powder inhalation (DPI) aerosol, which may be formulated as a dry powder of the active compounds with or without additional excipients.

Suitable topical formulations of the active compounds include transdermal devices, aerosols, creams, ointments, lotions, dusting powders, and the like.

In practical use, the active compounds can be combined in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, capsules and tablets, with the solid oral preparations being preferred over the liquid preparations. Because of their ease of administration, tablets and capsules represent the most advantageous oral dosage unit form in which case solid pharmaceutical carriers are obviously employed. If desired, tablets may be coated by standard aqueous or nonaqueous techniques.

In addition to the common dosage forms set out above, the one or more of the active compounds may also be administered by controlled release means and/or delivery devices such as those described in U.S. Patent Nos. 3,845,770; 3,916,899; 3,536,809; 3,598,123; 3,630,200 and 4,008,719.

Pharmaceutical compositions of the present invention suitable for oral administration may be presented as discrete units such as capsules, cachets or tablets each containing a predetermined amount of the active ingredients, as a powder or granules or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or molding, optionally with one or more accessory ingredients. Compressed tablets may be prepared by compressing in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Desirably, each tablet contains from about 1 mg to about 500 mg of each of the active ingredient and each cachet or capsule contains from about 1 to about 500 mg of each of the active ingredient.

The amounts of PGD2 antagonist and the other active ingredient(s) to achieve therapeutic effects will vary, depending on the activities of the specific compounds used, the specific disease to be treated, the severity of the disease, and the conditions of the patients to be treated. The dose for each active compound may be one usually used when the drug is administered alone, or it may be lower than such usual dose as the combination of the active ingredients may be synergistic for the treatment of the target diseases. Generally the dose may be between about 1 and about 1000 milligrams of each compound administered in a dose. The compounds may be combined in a single dosage formulation, or may be administered in separate dosage forms, and these may be solid (such as tablets, capsules, sachets and the like), liquid (such as solutions or suspensions) or inhalation aerosols for either or both compounds. While the solid compounds will typically be administered orally, the liquids may be administered orally or by injection. Other dosage forms, such as suppositories, are also useful.

The weight ratio of the compound of the prostaglandin D2 antagonist to the second active ingredient may be varied and will depend upon the effective dose of each ingredient. Generally, an effective dose of each will be used. Thus, for example, when a PGD2 antagonist is combined with an antihistamine the weight ratio of the PGD2 antagonist to the antihistamine will generally range from about 1000:1 to about 1:1000, preferably about 200:1 to about 1:200. Combinations of a PGD2 antagonist and a leukotriene antagonist will generally also be within the aforementioned range, but in each case, an effective dose of each active ingredient should be used.

### EXAMPLE 1

### Guinea Pig Allergic Rhinitis Model

Male Hartley guinea pigs (250-500 g) purchased from Charles River (St-Constant, Qc, Canada) were used. They were housed in a temperature and humidity controlled environment, in groups of four or five with food and water available *ad libitum.* Experimental procedures were approved by the Animal Care Committee at Merck Frosst Centre for Therapeutic Research, in accordance with the guidelines of the Canadian Council on Animal Care.

*Sensitization:* The animals were injected intraperitoneally with 0.5 ml of an ovalbumin solution (100 µg/ml) containing 100 mg/ml aluminum hydroxide in 0.9% saline. Another 0.5 ml (5 x 0.1 ml, subcutaneous.) of that solution was evenly distributed in the proximity of the lymph nodes (neck, axilla and inguinal regions). Experiments were conducted 2 weeks later.

*Measurements of intranasal pressure*: The animals were anaesthetized with sodium pentobarbital (40 mg/kg intraperitoneal + 10 mg/kg subcutaneous) and were placed in a supine position. The left jugular vein and the right carotid artery were cannulated with PE-50 tubing to allow drug injections and recording of heart rate and blood pressure, respectively. The trachea was exposed, sectioned and its lung-side was cannulated with a polyethylene cannula to allow mechanical ventilation (Harvard respirator, Model 683) with room air at a tidal volume of 4 ml/stroke and a rate of 60 strokes per minute. The nasal side of the trachea was also cannulated and connected to a small animal respirator (Harvard respirator, Model 683). A fixed amount of room air (tidal volume of 4 ml/stroke and a rate of 70 strokes/min) was continuously insufflated in the nasal cavity. To prevent any pressure loss, the esophagus was ligated and the mouth was sealed with an adhesive agent (Vet bond, 3M).

Following the surgical preparation, gallamine (2 mg/kg, i.v.) was administered to suppress spontaneous breathing. A period of 10 minutes was allowed for stabilization of the animals and recording of the baseline values of intranasal pressure, heart rate and blood pressure. The changes in the intranasal pressure were monitored through a pressure transducer (Validyne DP-45, membrane 6-26, Validyne Corp., Northridge, CA) connected to a side arm of the nasal cannula. Values were recorded every 5 seconds using a data acquisition system (Modular Instruments, Malvern, Pa.). A nasal challenge was performed by delivering for 3 minutes an aerosol of ovalbumin 1% (or saline) into the nasal cavity via an ultrasonic nebulizer (AeroSonic model 5000D, DeVilbiss; Somerset PA) positioned between the respirator and the nasopharynx. The changes in intranasal pressure were recorded for 30 minutes following the nasal challenge, using the peak response.

The effects of test compounds were evaluated on the increased intranasal pressure following a nasal challenge with an aerosol of ovalbumin 1% for 3 minutes. The test compounds were freshly prepared in 0.9% saline and were injected intraperitoneally in a dosing volume of 1 ml/kg, 60 minutes prior to the induction of the nasal antigen challenge. The changes in intranasal pressure were recorded for the 30 minutes following the nasal challenge, using the peak response. The test compounds are: mepyramine (a histamine H1 antagonist, 5 mg/kg); Compound I (Example 4, 1 mg/kg); mepyramine (5 mg/kg) + Compound I (0.3 mg/kg); and mepyramine (5 mg/kg) + Compound I (1 mg/kg).

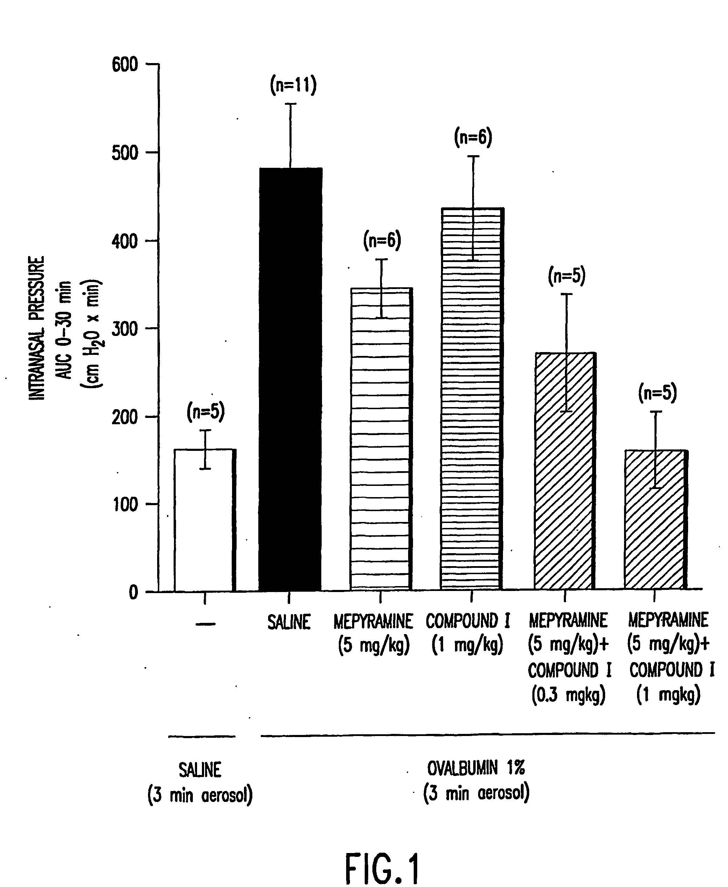

The results are shown graphically in FIG. 1. The area under the response curve was calculated from 0 - 30 minutes following the nasal challenge and the results were expressed as mean ± SEM from n = 5-11 separate experiments. Statistical differences between groups were analysed by analysis of variance (ANOVA) with multiple comparison (Bonferroni). P ≤ 0.05 was considered statistically significant.

The delivery of an aerosol of ovalbumin into the nasal cavity of sensitized guinea pigs induced a significant increase in the intranasal pressure compared to that of saline. The single administration of mepyramine (5 mg/kg i.p.) or Compound I (1 mg/kg, i.p.) 60 minutes prior to the ovalbumin challenge had no significant effect on the increase in intranasal pressure. However, in similar experimental conditions, the increase in intranasal pressure produced by the aerosol of ovalbumin was significantly blocked by the combination of mepyramine (5 mg/kg i.p.) and Compound I (0.3 or 1 mg/kg, i.p).

### EXAMPLE 2

### Nasal Airway Resistance In Conscious Sheep Following PGD2 or LTD4 or PGD2 + LTD4 Challenge

Nasal airway resistance (NAR) in sheep was measured using a modified mask rhinomanometry technique. Rhinometry in small experimental animals have been described in Kaise T, Ukai K, Pedersen OF, Sakakura Y, Accuracy of measurement of acoustic rhinometry applied to small experimental animals Am. J. Rhinology 1999, 13: 125-129 and Ohkawa C, Ukai K, Miyahara Y, Sakakura Y, Acoustic rhinometry evaluation of nasal response to histamine and antigen in guinea pigs. Am. J. Rhinology 1999, 13: 67-71. The allergic sheep model used is well in the art; see for example, Abraham, W.M., A. Ahmed, T. Ahmed, N. Atkins, and Andersson, Pharmacological evaluation of an allergic rhinitis model in sheep. Am. J. Respir. Crit. Care Med. 1998, 157: A616 and Lambrou, P. , Y. Botvinnikova, A. Ahmed and W.M. Abraham, Early and late mediator and cellular responses after nasal allergen provocation in the sheep model of allergic rhinitis. Am. J. Respir. Crit. Care Med. 2000, 161: A324.

The mediators in phosphate buffered saline were delivered to each nostril via an atomizer as follows: 40 nasal sprays of 0.05% PGD₂; 40 nasal sprays of 0.01% LTD4; or 40 nasal sprays of 0.05% PGD₂ + 40 nasal sprays of 0.01% LTD4.

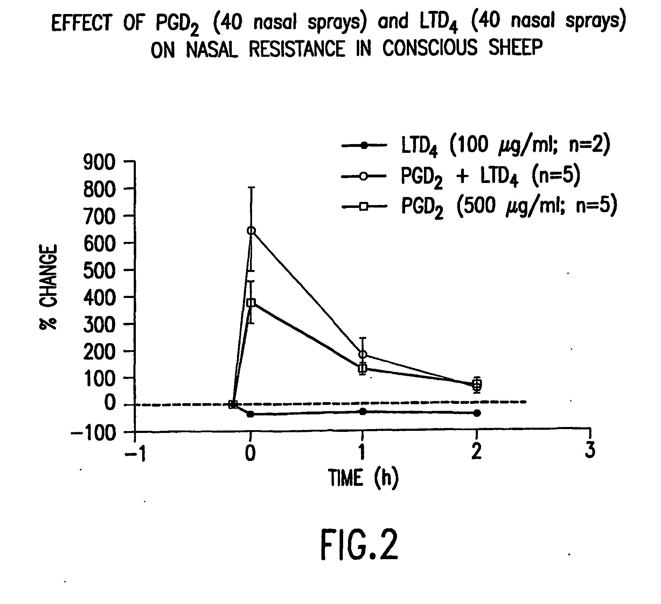

The results shown in FIG. 2 indicate that the combination of LTD4 and PGD2 produced a greater than additive effect in nasal resistance than either agent alone. These results support the combination of DP receptor antagonists with LTD4 antagonists alone or in combination with H1 antagonists for use in the treatment of various allergic conditions such as rhinitis, sinusitis, conjunctivitis, asthma and related respiratory diseases.

### EXAMPLE 3

### Assays for identifying and evaluating PGD2 receptor antagonists

### A) Radioligand binding assays using membranes from cells that express recombinant DP.

Radioligand binding assays are conducted essentially as previously described (Abramovitz et al., Biochem. Biophys. Acta 1483- 2, 285-293, 2000). HEK293(EBNA) cells expressing DP are grown in supplemented DMEM complete medium at 37°C in a humidified atmosphere of 6 % CO₂ in air, and then harvested. Cells are disrupted by nitrogen cavitation at 800 psi for 30 min. on ice in the presence of protease inhibitors (2 mM phenylmethylsulfonylfluoride, 10 µM E-64, 100 µM leupeptin and 0.05 mg/mL pepstatin). Membranes are prepared by differential centrifugation (1000 x g for 10 min, then 160,000 x g for 30 min, all at 4°C). The 160,000 x g pellets are resuspended in 10 mM HEPES/KOH (pH 7.4) containing 1 mM EDTA at approximately 5-10 mg/mL protein by Dounce homogenization (Dounce A; 10 strokes), frozen in liquid nitrogen and stored at -80°C. DP receptor binding assays are performed in a final incubation volume of 0.2 mL in 10 mM HEPES/KOH (pH 7.4), containing 1 mM EDTA, 10 mM MnCl₂, 0.7 nM [³H]PGD₂ (115-200 Ci/mmol). The reaction was initiated by addition of 30-60 µg membrane protein from the 160,000 x g fraction. Test compounds are added in dimethylsulfoxide (Me₂SO) at 1 % (v/v) in all incubations. Non-specific binding was determined in the presence of 1-10 µM of non-radioactive PGD₂. Incubations are conducted for 60 min. at room temperature. Incubations are terminated by rapid filtration at 4°C. Radioactivity bound to the individual filters is determined by scintillation counting. Maximum specific binding is defined as the total binding minus the non-specific binding. Specific binding is determined at each concentration of test compound and is expressed as a percentage of the maximum specific binding.

### B) Reporter-gene based functional assays using cells that express recombinant DP.

DP antagonists can be identified using reporter-gene (CRE-SEAP) assays using HEK293(EBNA) cells expressing recombinant DP (DP/293E/CRE-SEAP cells) Assays are performed in two steps: SEAP generation followed by measurement of SEAP activity. The SEAP generation step is conducted in a final volume of 100 or 200 µL of Ham's F12 supplemented with 0.1 % (v/v) bovine calf serum (BCS) and 0.01 % pluronic acid (F68) (HBF medium) containing 10⁴-10⁵ DP/293E/CRE-SEAP cells. Cells are pre-incubated for 15 min. at 37°C with the test compound added in Me₂SO at 0.5-1 % (v/v). Following preincubation with antagonist, the reaction is initiated by addition of the appropriate agonist e.g. PGD₂, added in Ham's F12 or Me₂SO. The samples are incubated for 7 hrs or overnight at 37°C. At the end of the incubation an aliquot of the assay medium is removed and mixed with an equal volume of substrate solution [1 M diethanolamine (pH 9.8) containing 10 mM L-homoarginine, 2 mM MgCl₂ and 20 mM pNPP (p-nitrophenylphosphate)]. SEAP activity is subsequently measured by following the hydrolysis of the substrate pNPP by monitoring changes in absorbance at 405 nm. DP antagonists inhibit PGD₂-induced SEAP activity.

### C. cAMP accumulation assays in cells expressing recombinant DP.

cAMP accumulation assays were conducted essentially as previously described (Wright et al., Eur. J. Pharmacol. 377, 101-115, 1999). HEK293(EBNA) cells expressing recombinant DP are harvested at 60-80% confluence by resuspension in enzyme-free cell-dissociation buffer and washed in phosphate-buffered saline by centrifugation (300 x g, 6 min. room temperature). The cells are then washed in Hank's balanced salt solution (HBSS) by centrifugation under the same conditions as described above. The generation of cAMP is performed in a final incubation volume of 0.2 mL HBSS containing 25 mM HEPES (pH 7.4), 500 µM IBMX or 100 µM Ro 20-1724 and 0.2-2 x 10⁵ DP expressing HEK293E cells. Samples are preincubated (10 min. at 37°C) with test compound added in Me₂SO at 0.5-1 % (v/v) in all incubations. Samples are then challenged with an appropriate concentration of an appropriate agonist e.g. PGD₂ added in Me₂SO at 0.5-1 % (v/v) and incubated for an additional 30 min. at 37°C. The reaction is terminated by boiling the samples for 3 min. and the cAMP content is measured by [¹²⁵I]cAMP SPA. A DP antagonist inhibits PGD₂-induced cAMP formation.

### D) cAMP accumulation assays in washed platelets.

Blood is collected from normal volunteers, who are free from medication for two weeks, by venous puncture of the antecubital vein in vacutainer tubes with no additive. The blood is immediately mixed with 10 % (v/v) citrate buffer (65 mM citric acid/85 mM sodium citrate/2 % glucose), subjected to centrifugation at 170 x g for 12 min. and the top layer removed (hPRP). Washed platelets are prepared by mixing hPRP with 30 % (v/v) citrate buffer and 50 % (v/v) 25 mM HEPES, HBSS without Ca²⁺ and Mg²⁺. The mixture is centrifuged at 800 x g for 12 min. and the pellet containing the platelet fraction washed two times by resuspension/centrifugation in 25 mM HEPES, HBSS without Ca²⁺ and Mg²⁺ containing 10% citrate buffer. The platelets are finally resuspended in 25 mM HEPES, HBSS without Ca²⁺ and Mg²⁺ at a concentration of 2.5 x 10⁸ cells/mL (hWP). hWP assays are conducted as follows: isobutylmethylxanthine (IBMX) (500 µM final concentration) is added in a 1:1000 ratio to hWP to prevent degradation of cAMP. Samples (100 µL) of either hWP are then preincubated (10 min. at 37°C) with test compound added in Me₂SO at 1 % (v/v). Samples are then challenged with an appropriate concentration of an appropriate agonist e.g. 300 nM PGD₂ added in Me₂SO at 1 % (v/v) and incubated for an additional 2 min. at 37°C. The reaction is then terminated by addition of 200 µL ice-cold ethanol to disrupt the cells and extract the cAMP. The samples are mixed thoroughly and centrifuged at 2000 x g for 15 min. at 4°C. Supernatant aliquots are removed and the ethanol removed by evaporation. cAMP is measured by [¹²⁵I]cAMP scintillation proximity assay (SPA) (Amersham) according to the manufacturers' instructions following reconstitution of the samples in SPA buffer. DP antagonists inhibit PGD₂-induced cAMP formation in hWP.

### EXAMPLE 4

### Preparation of 2-[(1R)-9-(4-chlorobenzyl)-8-((R)-methylsulfinyl)-2,3,4,9-tetrahydro-1H-carbazol-1-yl]acetic acid and 2-[(1R)-9-(4-chlorobenzyl)-8-((S)-methylsulfinyl)-2,3,4,9-tetrahydro-1H-carbazol-1-yl]acetic acid (Compound I)

### Step a(1). 1-[2-(methylsulfanyl)phenyl]hydrazine

2-(Methylthio)aniline (30g, 215mmol) was dissolved in 2N HCl (215ml) and cooled to 0°C and a solution of NaNO₂ (16.3g, 237mmol) in 50ml water was added dropwise (maintaining the temperature below 5°C). After 10 min the solution was added portionwise to a solution of Na₂S₂O₄ (220g 85% pure, 1075mmol) in a biphasic mixture of 1200ml of ether and 1200 mL of water dropwise (maintaining the temperature below 5°C). After stirring for one hour at 0°C the mixture was warmed to room temperature and the pH set to 10 with 2N NaOH. The ether layer was separated and the aqueous layer washed once with ether. The combined organic layers were dried with sodium sulfate, the solvent removed and the product purified on silica with 25% ethyl acetate/hexane to provide 15.7g of the title compound (47%). ¹H NMR (400 MHz), DMSO, δ: 2.30 (s, 3H); 4.10 (s, 2H); 6.20 (s, 1H); 6.60 (t, 1H); 7.10 (m, 2H); 7.20 (d, 2H).

### Step a(2). 1-[2-(methylsulfanyl)phenyl]hydrazine hydrochloride

Bromothioanisole (414g, 2041mmol) was added dropwise to a suspension of Mg (54.6g, 2245mmol) in 1000ml tetrahydrofuran under N₂ (maintaining a gentle reflux). The mixture was refluxed for 2 hours and cooled to -78°C. Solid di-tert-butyl azodicarboxylate (470g, 2041mmol) was added portionwise maintaining the temperature below -50°C. The mixture was stirred for 10 min, warmed to -30°C and quenched with 1 eq of acetic acid, 1000ml of water and 1000ml of ether. After agitation the ether layer was collected and dried with sodium sulfate. The solvent was removed and the crude di(*tert*-butyl) 1-[2-(methylsulfanyl)phenyl]-1,2-hydrazinedicarboxylate used as is in the next step.

Crude di(*tert*-butyl) 1-[2-(methylsulfanyl)phenyl]-1,2-hydrazine-dicarboxylate was dissolved in 8000ml of 1M HCl in ether. HCl gas was bubbled through the mixture for approximately 10 min every 2 hours, over a period of 6 hours. The mixture was stirred overnight and a precipitate formed. The solid was collected by filtration and washed with ether to provide 262g of the title compound (69% from bromothioanisole). ¹H NMR (400 MHz), DMSO, δ: 2.40 (s, 3H); 7.00 (m, 2H); 7.20 (t, 1H); 7.35 (d, 1H); 7.70 (s, 1H); 10.15 (s, 3H).

### Step b. ethyl 2-[8-(methylsulfanyl)-2,3,4,9-tetrahydro-1H-carbazol-1-yl]acetate

Method A. 1-[2-(methylsulfanyl)phenyl]hydrazine (15.7g, 102mmol) and ethyl 2-cyclohexanoneacetate (18.7g, 102mmol) were dissolved in 300ml isopropanol containing leq HCl. The mixture was refluxed overnight under nitrogen then cooled to room temperature. The solvent was stripped and the residue partitioned between 300ml water and 300 ml of dichloromethane. The water layer was washed with dichloromethane, and the organic layers were combined, dried with sodium sulfate and the solvent removed. The mixture was purified on silica with 5% ethyl acetate/toluene to provide 14.2g (46%) of the title compound.

Method B. 1-[2-(methylsulfanyl)phenyl]hydrazine hydrochloride (50g, 262mmol) and ethyl 2-cyclohexanoneacetate (48.3g, 262mmol) were dissolved in 1300ml isopropanol. The mixture was refluxed overnight under nitrogen then cooled to room temperature. The solvent was stripped and the residue partitioned between 1300ml water and ethyl acetate. The water layer was washed with ethyl acetate, and the organic layers were combined, dried with sodium sulfate and the solvent removed. The mixture was purified on silica with 2.5% ethyl acetate/toluene to provide 42g crude title compound.
¹H NMR (400 MHz), DMSO, δ: 1.20 (t, 3H); 1.60 (m, 1H); 1.70 (m, 1H); 1.80 (m, 1H); 1.95 (m, 1H); 2.30-2.45 (m, 1H); 2.45 (s, 3H);2.55 (t, 2H); 3.20 (dd, 1H); 3.30 (m, 1H); 4.10 (q, 2H); 6.90 (t, 1H); 7.00 (d, 1H); 7.25 (d, 1H); 10.60 (s, 1H).

### Step c. 2-[8-(methylsulfanyl)-2,3,4,9-tetrahydro-1H-carbazol-1-yl]acetic acid (5)

Crude product of step b (42g) was dissolved in 400ml of tetrahydrofuran and methanol (1:1) and 207ml of 2N LiOH was added thereto. The mixture was refluxed for 30 min and cooled to room temperature. The organic solvents were removed and 800ml of 1N HCl and 800 ml of ethyl acetate were added. The layers were separated and the aqueous layer washed with ethyl acetate. The combined organic layers were dried with sodium sulfate and the solvent removed. The resulting solid was triturated with 200ml 5% ether/hexane to provide 30.4g of the title compound. ¹H NMR (400 MHz), DMSO, δ: 1.60 (m, 1H); 1.70 (m, 1H); 1.80 (m, 1H); 1.95 (m, 1H); 2.30 (q, 1H); 2.45 (s, 3H); 2.55 (s (broad), 2H); 3.10 (dd, 1H); 3.25 (m, 1H); 6.90 (t, 1H); 7.00 (d, 1H); 7.25 (d, 1H); 10.55 (s, 1H); 12.25 (s, 1H).

### Step d. 2-[9-(4-chlorobenzyl)-8-(methylsulfanyl)-2,3,4,9-tetrahydro-1H-carbazol-1-yl]acetic acid

The product of step c in 100ml DMF (30.4g, 110mmol) was added to a suspension of a 60% NaH dispersion in mineral oil (11g, 276mmol) in 500ml DMF at -78°C under N2. The mixture was warmed to room temperature, stirred for 30 min and then cooled to -78°C. A solution of 220 mmol of 4-chlorobenzyl chloride in 100ml of dimethylformamide was added thereto, and the mixture warmed to room temperature and stirred for 4 hours. 500ml of 1N HCl and 500ml isopropyl acetate were added. The layers were separated and the organic layer washed 2 times with water. The organic layer was dried with sodium sulfate and the solvent removed. The resulting residue was purified on a plug of silica to provide 35g of the title compound.
¹H NMR (400 MHz), DMSO, δ: 1.60-1.90 (m, 4H); 2.30 (s, 3H); 2.35-2.40 (m, 2H); 2.60 (m, 1H); 2.85 (m, 1H); 3.20 (d, 1H); 5.50 (d, 1H); 6.00 (d, 1H); 6.70 (d, 2H); 7.05 (m, 2H); 7.30 (m, 3H); 12.30 (s, 1H).

### Step e. 2-[(1R)-9-(4-chlorobenzyl)-8-(methylsulfanyl)-2,3,4,9-tetrahydro-1H-carbazol-1-yl]acetic acid

The racemic acid of step d (35 g, 91.3 mmol) was dissolved in dry ethanol (900 mL) and heated to reflux. (R)-(+)-1-(1-naphthyl)ethylamine (15.64 g, 91.3 mmol, 1 eq) was added and the reaction mixture was stirred at 80°C for 30 min, the allowed to cool slowly to room temperature. Resulting suspension was stirred for 16 hours.

The salt was filtered and air dried for 2 hours to yield 15.2 g of white solid. The latter was recrystallized in ethanol (700 mL) to afford 13.4 g of salt. It was suspended in methanol (200 mL) and acidified with 3N HCl (11.5 mL). Resulting solution was concentrated to dryness and residue was partitioned in 1:1 ethyl acetate/H₂O. Organic fraction was dried with Na₂SO₄, and concentrated to give 9.4 g of solid.

The acid was analyzed by HPLC on chiralpak AD (250 x 4.6 mm). Elution was performed with a mixture of 15% 2-propanol in hexane and 0.2% acetic acid. A retention time of 8.4 min. was observed and the acid was obtained in 99.7% ee.

### Step f. methyl 2-[(1R)-9-(4-chlorobenzyl)-8-(methylsulfanyl)-2,3,4,9-tetrahydro-1H-carbazol-1-yl]acetate

The acid of step e (8.0 g, 20.0 mmol) was dissolved in acetone (250 mL) and treated with diazomethane (approximately 2M solution in diethyl ether) until yellow color remained. Excess CH₂N₂ was quenched with acetic acid, and the reaction mixture was concentrated to dryness to afford a yellow oil (8.3 g). (100%).
¹H NMR (acetone d₆) d 7.37 (d, 1H), 7.26 (d, 2H), 7.15 (d, 1H), 7.03 (t, 1H), 6.78 (d, 2H), 6.2 (d, 1H), 5.65 (d, 1H), 3.65 (s, 3H), 3.4-3.3 (m, 1H), 2.81-2.75 (m, 1H), 2.66-2.5 (m, 3H), 2.3 (s, 3H), 1.93-1.75 (m, 4H).

### Step g. methyl 2-[(1R)-9-(4-chlorobenzyl)-8-((S)-methylsulfinyl)-2,3,4,9-tetrahydro-1H-carbazol-1-yl]acetate and methyl 2-[(1R)-9-(4-chlorobenzyl)-8-((R)-methylsulfinyl)-2,3,4,9-tetrahydro-1H-carbazol-1-yl]acetate

The sulfide of step f (8.3 g, 20.0 mmol) was dissolved in dichloromethane (300 mL) and mCPBA (4.0 g @ 85%, 20.0 mmol, 1 eq) was added. The mixture was stirred at room temperature for 30 min, washed with saturated NaHCO3 (2x25 mL), dried with sodium sulfate, and concentrated to dryness to give 8.6 g of yellow foam.

The product was a mixture of two diastereomers which was separated by HPLC on Zorbax Pro 10 process column, eluting with 25% 2-propanol in hexane.

3.46 g of the less polar diastereomer and 2.72 g of more polar diastereomer were recovered.
¹H NMR (acetone d6)
Less polar compound: d 7.8 (d, 1H), 7.66 (d, 1H), 7.35-7.25 (m, 3H), 6.8 (d, 2H), 5.78 (d, 1H), 5.41 (d, 1H), 3.6 (s, 3H), 3.43-3.35 (m, 1H), 2.9-2.6 (m, 2H), 2.52 (d, 2H), 2.3 (s, 3H), 2.0 - 1.85 (m, 4H).
More polar compound: d 7.77 (d, 1H), 7.65 (d, 1H), 7.35-7.25 (m, 3H), 6.75 (d, 2H), 5.58 (d, 1H), 5.42 (d, 1H), 3.65 (s, 3H), 3.4 - 3.3 (m, 1H), 2.9 - 2.56 (m, 4H), 2.54 (s, 3H), 2.0-1.85 (m, 4H).

### Step h(1). 2-[(1R)-9-(4-chlorobenzyl)-8-((S)-methylsulfinyl)-2,3,4,9-tetrahydro-1H-carbazol-1-yl]acetic acid

The less polar ester of step g (2.36 g, 5.5 mmol) was dissolved in 25 mL of THF:MeOH (3:1 mixture) and 2N LiOH (7.1 mmol, 1.3 eq) was added. The reaction mixture was stirred at room temperature for 2 hours and a white suspension was obtained. When acidified to pH 2 with 1N HCl the reaction mixture became clear. After stirring at room temperature for 1 hr, the acid product precipitated. The solid was filtered and washed with small volume of ethyl acetate to afford 2.1 g (92%) of the title compound.
¹H NMR (DMSO d6): d 7.7 (d, 1H), 7.65 (d, 1H), 7.35 (d, 2H), 7.27 (t, 1H), 6.72 (d, 2H), 5.62 (d, 1H), 5.38 (d, 1H), 2.8 (d, 1H), 2.65-2.5 (m, 1H), 2.38-2.28 (m, 2H), 2.35 (s, 3H), 1.92-1.75 (m, 4H).
Optical rotation: +121.3° (c=0.39 in methanol).

### Step h(2) 2-[(1R)-9-(4-chlorobenzyl)-8-((R)-methylsulfinyl)-2,3,4,9-tetrahydro-1H-carbazol-1-yl]acetic acid

The more polar ester of step g (1.6 g, 3.7 mmol) was dissolved in 15 mL of THF:MeOH (3:1 mixture) and 2N LiOH (4.8 mmol, 1.3 eq) was added. The reaction mixture was stirred at room temperature for 2 hours and a white suspension was obtained. When acidified to pH 2 with 1N HCl the reaction mixture became clear. After stirring at room temperature for 1 hr, acid product precipitated. The solid was filtered and washed with small volume of ethyl acetate to afford 1.37 g (89%) of the title compound.
¹H NMR (DMSO d6): d 7.66 (d, 1H), 7.63 (d, 1H), 7.34 (d, 2H), 7.28 (t, 1H), 6.69 (d, 2H), 5.42 (d, 1H), 5.24 (d, 1H), 3.2 (d, 1H), 2.8 (d, 1H), 2.68-2.54 (m, 2H), 2.58 (s, 3H), 2.47 - 2.39 (m, 1H), 1.9 - 1.75 (m, 4H).
Optical rotation: -231.9 (c=0.3 in methanol).

### REFERENCE EXAMPLE

### The compound of Example 4, step d may also be prepared as follows: Step a. diphenylmethanone N-[2-(methylsulfanyl)phenyl]hydrazone

1-[2-(Methylsulfanyl)phenyl]hydrazine hydrochloride (30g, 148mmol) was dissolved in 300ml DMF and benzophenone imine (26.7g, 148mmol) was added dropwise over 5 min. The mixture was stirred for 1 hour and 300ml ether and 300ml of water were added. The layers were separated and the organic layer washed twice with brine. The organic layer was dried with sodium sulfate and the solvent removed. The residue with triturated with hexane to obtain 38.5g of title compound (containing 18 % benzophenone). ¹H NMR (400 MHz), DMSO, δ: 2.60 (s, 3H); 6.80 (t, 1H); 7.30-7.45 (m, 7H); 7.55 (d, 2H); 7.60 (t, 2H); 7.65 (s, 2H); 8.40 (s, 1H).

### Step b. diphenylmethanone N-(4-chlorobenzyl)-N-[2-(methylsulfanyl)phenyl]hydrazone

Diisopropylamine (29ml, 206mmol) was dissolved in 50 ml THF and cooled to 0°C. 76ml n-BuLi (2M in c-Hexane) was added dropwise and the solution was stirred for 30 min. This solution was then cannulated into a solution of 61.6g of the product of step a (containing 18% benzophenone) in 150ml THF at 0°C. The mixture was stirred at room temperature for 30min, cooled to 0°C and 4-bromobenzyl bromide (39.1g, 190.3mmol) in 50 ml THF was added. The mixture was stirred for 30 min and 200ml of NH₄Cl (sat) and ether were added. The layers were separated and the aqueous layer washed with ether. The combined organic layers were dried with sodium sulfate and the solvent removed. The residue was triturated with hexane to obtain 67g of the title compound. ¹H NMR (400 MHz), DMSO, δ: 2.35 (s, 3H); 4.40 (s, 2H); 6.80-7.00 (m, 6H); 7.10 (t, 3H); 7.30 (m, 5H); 7.40 (d, 2H); 7.50 (d, 2H).

### Step c. 2-[9-(4-chlorobenzyl)-8-(methylsulfanyl)-2,3,4,9-tetrahydro-1H-carbazol-1-yl]acetic acid

The product of step b (84.6g, 191mmol) and ethyl 2-cyclohexanoneacetate (35.2g, 191mmol) were dissolved in 850ml ethanol and p-toluenesulfonic acid (72.8g, 381mmol) was added. The mixture was refluxed for 3 hours, cooled to room temperature and the solvent stripped. 1000ml ether and 1000ml of water were added. The layers were separated and the organic layer washed with brine, dried with sodium sulfate and the solvent removed. The residue was purified on silica with 3% ethyl acetate/Hex. Crude Indole (43.6g) contained 12 % benzophenone and 22% of ethyl 2-[9-(4-chlorobenzyl)-2,3,4,9-tetrahydro-1*H*-carbazol-1-yl]acetate. 43.4g of the crude mixture was dissolved in 500ml of THF and MeOH and 152ml of 2N LiOH was added. The mixture was refluxed for 30 min and cooled to room temperature. The organic solvents were removed and 800ml of 1N HCl and ethyl acetate added. The layers were separated and the aqueous layer washed with ethyl acetate. The combined organic layers were dried with sodium sulfate and the solvent removed. The resulting solid was purified on a short silica column with 25% ethyl acetate/toluene/ 1% acetic acid to provide 32g of the title compound, contaminated with ethyl 2-[9-(4-chlorobenzyl)-2,3,4,9-tetrahydro-1*H*-carbazol-1-yl]acetate. ¹H NMR (400 MHz), DMSO, δ: 1.60-1.90 (m, 4H); 2.30 (s, 3H); 2.35-2.40 (m, 2H); 2.60 (m, 1H); 2.85 (m, 1H); 3.20 (d, 1H); 5.50 (d, 1H); 6.00 (d, 1H); 6.70 (d, 2H); 7.05 (m, 2H); 7.30 (m, 3H); 12.30 (s, 1H).

## Claims

1. A combination of a prostaglandin D2 antagonist and at least one other therapeutically active compound selected from histamine H1 antagonists and leukotriene D4 antagonists, for use in the treatment of an allergic condition.

2. A combination according to Claim 1 wherein said allergic condition is allergic rhinitis.

3. A pharmaceutical composition comprising an effective amount of a prostaglandin D2 antagonist and an effective amount of at least one other active ingredient selected from histamine H1 antagonist and leukotriene D4 antagonists, and a pharmaceutically acceptable carrier.

4. A pharmaceutical composition according to Claim 3 wherein said other active ingredient is a histamine H1 antagonist.

5. A pharmaceutical composition according to Claim 3 wherein said other active ingredient is a leukotriene D4 antagonist.

6. A pharmaceutical composition according to Claim 3 comprising an effective amount of a prostaglandin D2 antagonist and an effective amount of a histamine H1 antagonist and an effective amount of a leukotriene D4 antagonist, and a pharmaceutically acceptable carrier.

7. A pharmaceutical composition according to Claim 4 or Claim 6 wherein said histamine H1 antagonist is selected from loratadine, descarboethoxyloratadine, cetirizine, levocetirizine and fexofenadine.

8. A pharmaceutical composition according to Claim 5 or Claim 6 wherein said leukotriene D4 antagonist is selected from zafirlukast, montelukast and pranlukast.

9. A process for the preparation of a pharmaceutical composition which comprises combining a prostaglandin D2 antagonist with at least one other active ingredient selected from histamine H1 antagonists and leukotriene D4 antagonists, and a pharmaceutically acceptable carrier.

10. A pharmaceutical composition prepared by the process of Claim 9.

11. Use of a prostaglandin D2 antagonist and at least one other therapeutically active compound selected from histamine H I antagonists and leukotriene D4 antagonists for the manufacture of a medicament for the treatment of allergic conditions.

12. Use according to Claim 11 wherein said other therapeutically active compound is a histamine H1 antagonist.

13. Use according to Claim 11 wherein said other therapeutically active compound is a leukotriene D4 antagonist.

14. Use of a prostaglandin D2 antagonist and a histamine H1 antagonist and a leukotriene D4 antagonist for the manufacture of a medicament for the treatment of allergic conditions.

15. Use according to Claim 12, or Claim 14 wherein said histamine H1 antagonist is selected from loratadine, descarboethoxyloratadine, cetirizine, levocetirizine and fexofenadine.

16. Use according to Claim 13 or Claim 14 wherein said leukotriene D4 antagonist is selected from zafirlukast, montelukast and pranlukast.

17. Use according to any one of Claims 11 to 16 wherein said allergic condition is allergic rhinitis.

## Patentansprüche

1. Kombination eines Prostaglandin-D2-Antagonisten und mindestens einer anderen therapeutisch aktiven Verbindung, ausgewählt aus Histamin-H1-Antagonisten und Leukotrien-D4-Antagonisten, zur Verwendung bei der Behandlung eines allergischen Zustands.

2. Kombination nach Anspruch 1, wobei der allergische Zustand allergische Rhinitis ist.

3. Pharmazeutische Zusammensetzung, umfassend eine wirksame Menge eines Prostaglandin-D2-Antagonisten und eine wirksame Menge von mindestens einem anderen aktiven Bestandteil, ausgewählt aus Histamin-H1-Antagonisten und Leukotrien-D4-Antagonisten, und einen pharmazeutisch annehmbaren Träger.

4. Pharmazeutische Zusammensetzung nach Anspruch 3, wobei der andere aktive Bestandteil ein Histamin-H1-Antagonist ist.

5. Pharmazeutische Zusammensetzung nach Anspruch 3, wobei der andere aktive Bestandteil ein Leukotrien-D4-Antagonist ist.

6. Pharmazeutische Zusammensetzung nach Anspruch 3, umfassend eine wirksame Menge eines Prostaglandin-D2-Antagonisten und eine wirksame Menge eines Histamin-H1-Antagonisten und eine wirksame Menge eines Leukotrien-D4-Antagonisten und einen pharmazeutisch annehmbaren Träger.

7. Pharmazeutische Zusammensetzung nach Anspruch 4 oder Anspruch 6, wobei der Histamin-H1-Antagonist aus Loratadin, Descarboethoxyloratadin, Cetirizin, Levocetirizin und Fexofenadin ausgewählt ist.

8. Pharmazeutische Zusammensetzung nach Anspruch 5 oder Anspruch 6, wobei der Leukotrien-D4-Antagonist aus Zafirlukast, Montelukast und Pranlukast ausgewählt ist.

9. Verfahren zur Herstellung einer pharmazeutischen Zusammensetzung, welches das Kombinieren eines Prostaglandin-D2-Antagonisten mit mindestens einem anderen aktiven Bestandteil, ausgewählt aus Histamin-H1-Antagonisten und Leukotrien-D4-Antagonisten, und einem pharmazeutisch annehmbaren Träger umfasst.

10. Pharmazeutische Zusammensetzung, hergestellt nach dem Verfahren nach Anspruch 9.

11. Verwendung eines Prostaglandin-D2-Antagonisten und mindestens einer anderen therapeutisch aktiven Verbindung, ausgewählt aus Histamin-H1-Antagonisten und Leukotrien-D4-Antagonisten, zur Herstellung eines Medikaments zur Behandlung allergischer Zustände.

12. Verwendung nach Anspruch 11, wobei die andere therapeutisch aktive Verbindung ein Histamin-H1-Antagonist ist.

13. Verwendung nach Anspruch 11, wobei die andere therapeutisch aktive Verbindung ein Leukotrien-D4-Antagonist ist.

14. Verwendung eines Prostaglandin-D2-Antagonisten und eines Histamin-H1-Antagonisten und eines Leukotrien-D4-Antagonisten zur Herstellung eines Medikaments zur Behandlung allergischer Zustände.

15. Verwendung nach Anspruch 12 oder Anspruch 14, wobei der Histamin-H1-Antagonist aus Loratadin, Descarboethoxyloratadin, Cetirizin, Levocetirizin und Fexofenadin ausgewählt ist.

16. Verwendung nach Anspruch 13 oder Anspruch 14, wobei der Leukotrien-D4-Antagonist aus Zafirlukast, Montelukast und Pranlukast ausgewählt ist.

17. Verwendung nach irgendeinem der Ansprüche 11 bis 16, wobei der allergische Zustand allergische Rhinitis ist.

## Revendications

1. Combinaison d'un antagoniste de la prostaglandine D2 et d'au moins un autre composé thérapeutiquement actif choisi parmi les antagonistes de l'histamine H1 et les antagonistes du leucotriène D4, pour une utilisation dans le traitement d'une affection allergique.

2. Combinaison selon la revendication 1, dans laquelle ladite affection allergique est la rhinite allergique.

3. Composition pharmaceutique comprenant une quantité efficace d'un antagoniste de la prostaglandine D2 et d'une quantité efficace d'au moins un autre ingrédient actif choisi parmi un antagoniste de l'histamine H1 et les antagonistes du leucotriène D4 et un transporteur pharmaceutiquement acceptable.

4. Composition pharmaceutique selon la revendication 3, dans laquelle ledit autre ingrédient actif est un antagoniste de l'histamine H1.

5. Composition pharmaceutique selon la revendication 3, dans laquelle ledit autre ingrédient actif est un antagoniste du leucotriène D4.

6. Composition pharmaceutique selon la revendication 3, comprenant une quantité efficace d'un antagoniste de la prostaglandine D2, et une quantité efficace d'un antagoniste de l'histamine H1, et une quantité efficace d'un antagoniste du leucotriène D4 et un transporteur pharmaceutiquement acceptable.

7. Composition pharmaceutique selon la revendication 4 ou la revendication 6, dans laquelle ledit antagoniste de l'histamine H1 est choisi parmi la loratadine, la descarboéthoxyloratadine, la cétirizine, la lévocétirizine et la fexofénadine.

8. Composition pharmaceutique selon la revendication 5 ou la revendication 6, dans laquelle ledit antagoniste du leucotriène D4 est choisi parmi le zafirlukast, le montelukast et le pranlukast.

9. Procédé pour la préparation d'une composition pharmaceutique qui comprend la combinaison d'un antagoniste de la prostaglandine D2 avec au moins un autre ingrédient actif choisi parmi les antagonistes de l'histamine H1 et les antagonistes du leucotriène D4 et un transporteur pharmaceutiquement acceptable.

10. Composition pharmaceutique préparée par le procédé selon la revendication 9.

11. Utilisation d'un antagoniste de la prostaglandine D2 et d'au moins un autre composé thérapeutiquement actif choisi parmi les antagonistes de l'histamine H1 et les antagonistes du leucotriène D4 pour la fabrication d'un médicament pour le traitement d'affections allergiques.

12. Utilisation selon la revendication 11, dans laquelle ledit autre composé thérapeutiquement actif est un antagoniste de l'histamine H1.

13. Utilisation selon la revendication 11, dans laquelle ledit autre composé thérapeutiquement actif est un antagoniste du leucotriène D4.

14. Utilisation d'un antagoniste de la prostaglandine D2 et d'un antagoniste de l'histamine H1 et d'un antagoniste du leucotriène D4 pour la fabrication d'un médicament pour le traitement d'affections allergiques.

15. Utilisation selon la revendication 12 ou la revendication 14, dans laquelle ledit antagoniste de l'histamine H1 est choisi parmi la loratadine, la descarboéthoxyloratadine, la cétirizine, la lévocétirizine et la fexofénadine.

16. Utilisation selon la revendication 13 ou la revendication 14, dans laquelle ledit antagoniste du leucotriène D4 est choisi parmi le zafirlukast, le montelukast et le pranlukast.

17. Utilisation selon l'une quelconque des revendications 11 à 16, dans laquelle ladite affection allergique est la rhinite allergique.
